(19) Europäisches Patentamt
European Patent Office
Office européen des brevets

(11) Veröffentlichungsnummer: **0 379 081 A2**

(12) **EUROPÄISCHE PATENTANMELDUNG**

(21) Anmeldenummer: **90100611.4**

(22) Anmeldetag: **12.01.90**

(51) Int. Cl.[5]: **C07D 303/14, C07D 301/02, C07C 49/825, C07F 7/18**

(30) Priorität: **18.01.89 DE 3901327**

(43) Veröffentlichungstag der Anmeldung: **25.07.90 Patentblatt 90/30**

(84) Benannte Vertragsstaaten: **AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Zierke, Thomas, Dr.**
**Bahnhofstrasse 10**
**D-6737 Boehl-Iggelheim(DE)**
Erfinder: **Sauter, Hubert, Dr.**
**Neckarpromenade 20**
**D-6800 Mannheim 1(DE)**
Erfinder: **Schulz, Guenter, Dr.**
**Carl-Bosch-Strasse 96**
**D-6700 Ludwigshafen(DE)**
Erfinder: **Will, Wolfgang, Dr.**
**Im Buegen 12**
**D-6719 Kirchheim(DE)**

(54) **Verfahren zur Herstellung von in 2-Position Z-substituierten 1-Hydroxymethyl-oxiranen.**

(57) Verfahren zur Herstellung von in 2-Position Z-substituierten 1-Hydroxymethyl-oxiranen der allgemeinen Formel I und ent-I,

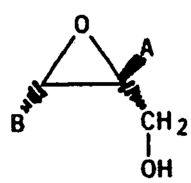

(I)

und

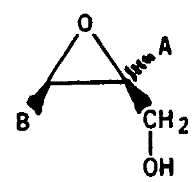

(ent-I)

in der die Substituenten
A, B unabhängig voneinander $C_1$-$C_4$-Alkyl, Phenyl, Naphthyl, Diphenyl oder durch ein bis drei Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl
bedeuten, indem man Ketone der allgemeinen Formel II

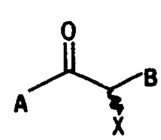

(II),

in der A und B die oben genannten Bedeutungen haben und X für eine unter den Reaktionsbedingungen eliminierbare Gruppe steht, mit Trimethylsulfoniummethylsulfat und einer geeigneten Base oder mit Dimethyloxosulfonium-methylid umsetzt, sowie neue Ketone der allgemeinen Formel II'

EP 0 379 081 A2

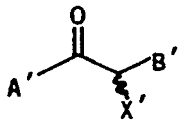

(II'),

in der die Substituenten
A' Phenyl oder 4-Fluorphenyl,
B' 2-Fluorphenyl, 2-Chlorphenyl, 2-Bromphenyl oder 2-Trifluormethylphenyl und
X' Hydroxy oder eine Abgangsgruppe
bedeuten, mit der Maßgabe, daß B' nicht für 2-Chlorphenyl steht, wenn A' Phenyl bedeutet.

## Verfahren zur Herstellung von in 2-Position Z-substituierten 1-Hydroxymethyl-oxiranen

Die vorliegende Erfindung betrifft ein Verfahren zur Herstellung von in 2-Position Z-substituierten 1-Hydroxymethyl-oxiranen aus entsprechenden Ketonen, die in $\alpha$-Position zur Carbonylfunktion eine unter den Reaktionsbedingungen eliminierbare Gruppe tragen, durch Umsetzung mit Trimethylsulfonium-methylsulfat und einer geeigneten Base oder mit Dimethyloxosulfonium-methylid, sowie neue Ketone.

Aus der EP-A-94 564 ist bekannt, daß man Hydroxymethyloxirane, z.B. durch Oxidation von Propenen in Allylposition und anschließende Epoxidierung erhält.

Aus der DE-A-3601927 ist ferner ein Verfahren zur Herstellung von Hydroxymethyloxiranen aus $\alpha,\beta$-ungesättigten Acroleinen durch alkalische Epoxidierung mit $H_2O_2$ und anschließende Reduktion der Aldehydfunktion bekannt.

Diese Verfahren waren noch verbesserungsbedürftig.

U.a. aus J. Chem. Soc. 223-225 (1935) und Bull. Chem. Soc. Jpn. 60, 4363-4368 (1987) sind Ketone des Typs II′ bekannt.

Der vorliegenden Erfindung lag daher die Aufgabe zugrunde, ein neues und verbessertes Verfahren zur Herstellung von Hydroxymethyloxiranen zu finden, ferner neue Ketone als Zwischenprodukte zu erhalten, die zur Durchführung des Verfahrens zur Herstellung der Hydroxymethyloxirane verwendet werden können.

Demgemäß wurde ein neues und verbessertes Verfahren zur Herstellung von in 2-Position Z-substituierten 1-Hydroxymethyl-oxiranen der allgemeinen Formeln I und ent-I,

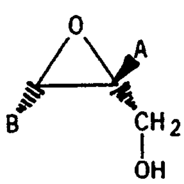

(I)

und

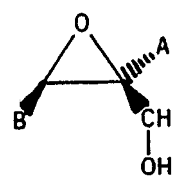

(ent-I)

in der die Substituenten

A, B unabhängig voneinander $C_1$-$C_4$-Alkyl, Phenyl, Naphthyl, Diphenyl oder durch ein bis drei Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl

bedeuten, gefunden, welches dadurch gekennzeichnet ist, daß man Ketone der allgemeinen Formel II

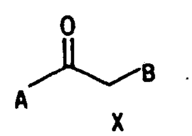

(II),

in der A und B die oben genannten Bedeutungen haben und X für eine unter den Reaktionsbedingungen eliminierbare Gruppe steht, mit Trimethylsulfonium-methylsulfat und einer geeigneten Base oder mit Dimethyloxosulfonium-methylid umsetzt, sowie neue Ketone der allgemeinen Formel II′

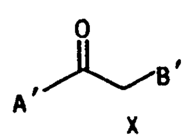

(II′),

in der die Substituenten

A′ Phenyl oder 4-Fluorphenyl,

B′ 2-Fluorphenyl, 2-Chlorphenyl, 2-Bromphenyl oder 2-Trifluormethylphenyl und

X′ Hydroxy oder eine Abgangsgruppe

bedeuten, mit der Maßgabe, daß B′ nicht für 2-Chlorphenyl steht, wenn A′ Phenyl bedeutet.

Die Zeichen

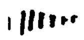 und 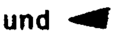

zeigen die räumliche Orientierung der Reste in einem chiralen Molekül an. Dazu wird in vorliegendem Fall der Oxiranring als in der Papierebene (Grundebene) liegend definiert. Reste, die sich an dem Zeichen

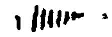

befinden, weisen nach unten aus der Papierebene hinaus, liegen also unterhalb der Papierebene, solche mit dem Zeichen

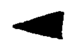

weisen nach oben aus der Papierebene hinaus, liegen also oberhalb der Papierebene. Das Zeichen

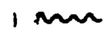

bedeutet, daß die Konfigiration an diesem Chiralitätszentrum unbestimmt ist und es sich in aller Regel um ein racemisches Gemisch handelt.

Bei der Verbindung mit der Bezeichnung ent-I handelt es sich um die Verbindung die zu I enantiomer ist.

Die Verbindungen 1 enthalten 2 chirale Zentren und werden im allgemeinen in Form von Diastereomerengemischen erhalten. Diese lassen sich durch Diastereomerentrennung, aufgrund ihrer unterschiedlichen physikalischen Eigenschaften z.B. durch Umkristallisation oder Säulenchromatographie, auch HPLC, in diastereomerenreine Enantiomerenpaare trennen. Bei der vorliegenden Erfindung wird bevorzugt nur ein diasteromeres Enantiomerenpaar gebildet. Die diastereomerenreinen Enantiomerenpaare können durch bekannte Methoden der Racematspaltung, wie z.B. durch Umsetzung mit einer chiralen Hilfskomponente, Trennung der entstandenen Diastereomeren nach den oben genannten Methoden und anschließende Abspaltung der Hilfskomponente oder durch Säulenchromatographie, auch HPLC, an chiralen Säulen (Säulenfüllmaterial z.B. Kieselgel dotiert mit einem chiralen nicht herauswaschbaren festen Hilfsstoff), in enantiomerenreine Verbindungen I überführt werden.

Das erfindungsgemäße Verfahren kann wie folgt durchgeführt werden:

Die Ketone II werden entweder

a) mit Trimethylsulfoniummethylsulfat und einer Base oder

b) mit Dimethyloxosulfoniummethylid zu den in 2-Position Z-substituierten 1-Hydroxymethyloxiranen der allgemeinen Formel I oder ent-I umgesetzt:

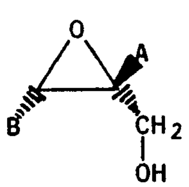

(I)

und

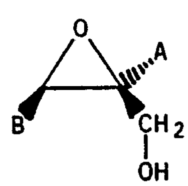

(ent-I)

a) Umsetzung mit Trimethylsulfonium-methylsulfat und einer Base:

Man legt in aller Regel das Trimethylsulfonium-methylsulfat und das Keton vor und gibt die Base zu. Die Reaktionstemperatur liegt im allgemeinen zwischen (-20) und +100 °C, bevorzugt zwischen 0 und 80 °C, besonders bevorzugt bei 20 bis 60 °C. Die Reaktion wird im allgemeinen bei Atmosphärendruck durchgeführt, ist jedoch auch bei Über- oder Unterdruck möglich.

Als Basen eignen sich starke anorganische Basen wie Natriumhydrid, Kaliumhydrid, Calciumhydrid, Kaliumhydroxid und Natriumhydroxid, bevorzugt Natriumhydrid, oder organische Basen wie Natriumamid,

Lithiumdiisopropylamid, Butyllithium, Methyllithium und Kalium-tert.-butylat, bevorzugt Natriumamid und Kalium-tert.-butylat.

Die Reaktion kann in Abwesenheit, vorzugsweise in Anwesenheit eines inerten Lösungsmittels durchgeführt werden. Als inerte Lösungsmittel eignen sich Ether, gesättigte Kohlenwasserstoffe, wie Pentan und Hexan, aromatische Kohlenwasserstoffe wie Toluol und Benzol, Nitrile, Sulfoxide oder auch tert. Alkohole, bevorzugt Dimethylsulfoxid, Acetonitril, tert.-Butanol, Toluol und Tetrahydrofuran, besonders bevorzugt tert.-Butanol oder Nitrile, wie Acetonitril.

Das Trimethylsulfonium-methylsulfat und dessen Herstellung ist aus Heterocycles 8, 397-401 (1977) bekannt.

b) Umsetzung mit Dimethyloxosulfonium-methylid:

In der Regel erzeugt man das Dimethyloxosulfonium-methylid in situ durch Reaktion von Trimethyloxosulfoniumiodid mit einer Base und gibt das Keton zu.

Die Reaktionstemperatur liegt im allgemeinen zwischen (-20) und +100 °C, vorzugsweise zwischen +20 und +80 °C, besonders bevorzugt bei 20 bis 60 °C. Die Reaktion wird im allgemeinen bei Atmosphärendruck durchgeführt, ist jedoch auch bei Über- oder Unterdruck möglich.

Die Reaktion kann in Abwesenheit, vorzugsweise in Anwesenheit eines inerten Lösungsmitels erfolgen. Als inerte Lösungsmittel eignen sich gesättigte Kohlenwasserstoffe wie Petrolether, Hexan und Cyclohexan, Ether, wie Diethylether und Tetrahydrofuran, aromatische Kohlenwasserstoffe wie Toluol und Benzol, Sulfoxide wie Dimethylsulfoxid, bevorzugt Tetrahydrofuran und Dimethylsulfoxid, besonders bevorzugt Dimethylsulfoxid.

Das Dimethyloxosulfoniummethylid und dessen Herstellung ist aus J.Am.Chem.Soc. 87, 1353-1364 (1965) bekannt. Andere Sulfonium- und Oxosulfonium-methylide lassen sich analog herstellen und könnten ebenfalls in der Reaktion eingesetzt werden.

Die Ketone II sind allgemein bekannte Verbindungen und können wie auch die neuen Ketone II′ nach allgemein bekannten Methoden der organischen Chemie erhalten werden. So erhält man beispielsweise α-Halogenketone durch Halogenierung von Ketonen (House, "Modern Synthetic Reactions", 2nd ed., pp 459-78). α-Silyloxyketone sind durch Silylierung der Hydroxyfunktion in α-Hydroxyketonen darstellbar (s. z.B. T.W. Greene, Protective Groups in Organic Synthesis, Wiley, 1981). Die neuen Ketone II′ sind Benzoinderivate. Die Benzoinkondensation ist besonders geeignet zur Darstellung von aromatischen α-Hydroxyketonen (Ide, Buck, Org. Reactions 4, 269-304 (1948)). In einer besonderen Ausführungsform können auch aliphatische Aldehyde oder Gemische von aliphatischen und aromatischen Aldehyden miteinander kondensiert werden (s. Stetter et al., Synthesis 733 (1976) und 403 (1988)), so daß α-Hydroxyketone allgemein zugänglich sind.

Im einzelnen haben die Substituenten A und B in den Formeln I und II unabhängig voneinander folgende Bedeutungen:

- unverzweigtes oder verzweigtes $C_1$-$C_4$-Alkyl, wie Methyl, Ethyl, n-Propyl, iso-Propyl, n-Butyl, iso-Butyl, sec.-Butyl und tert.-Butyl,
- Phenyl,
- Naphthyl, wie 1-Naphthyl und 2-Naphthyl, bevorzugt 2-Naphthyl,
- Diphenyl, wie o-Diphenyl, m-Diphenyl und p-Diphenyl,
- durch ein, zwei oder drei Halogenatome substituiertes Phenyl, bevorzugt durch ein oder zwei Halogenatome substituiertes Phenyl, wie 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2,3-Difluorphenyl, 2,4-Difluorphenyl, 2,5-Difluorphenyl, 2,6-Difluorphenyl, 3,4-Difluorphenyl, 3,5-Difluorphenyl, 2,3-Difluorphenyl, 2,4-Dichlorphenyl, 2,3-Dichlorphenyl, 2,5-Dichlorphenyl, 2,6-Dichlorphenyl, 3,4-Dichlorphenyl, 3,5-Dichlorphenyl, 2,3-Dibromphenyl, 2,4-Dibromphenyl, 2,5-Dibromphenyl, 2,6-Dibromphenyl, 3,4-Dibromphenyl, 3,5-Dibromphenyl, 2-Chlor-3-fluorphenyl, 3-Chlor-2-fluorphenyl, 2-Chlor-4-fluorphenyl, 4-Chlor-2-fluorphenyl, 2-Chlor-5-fluorphenyl, 5-Chlor-2-fluorphenyl, 2-Chlor-6-fluorphenyl, 3-Chlor-4-fluorphenyl, 4-Chlor-3-fluorphenyl, 3-Chlor-5-fluorphenyl, 2-Brom-3-fluorphenyl, 3-Brom-2-fluorphenyl, 2-Brom-4-fluorphenyl, 4-Brom-2-fluorphenyl, 2-Brom- 5-fluorphenyl, 5-Brom-2-fluorphenyl, 2-Brom-6-fluorphenyl, 3-Brom-4-fluorphenyl, 4-Brom-3-fluorphenyl, 3-Brom-5-fluorphenyl, 2-Brom-3-chlorphenyl, 3-Brom-2-chlorphenyl, 2-Brom-4-chlorphenyl, 4-Brom-2-chlorphenyl, 2-Brom-5-fluorphenyl, 5-Brom-2-chlorphenyl, 4-Brom-6-chlorphenyl, 3-Brom-4-chlorphenyl, 4-Brom-3-chlorphenyl, 3-Brom-5-chlorphenyl,
- durch ein, zwei oder drei $C_1$-$C_4$-Alkylgruppen substituiertes Phenyl,
- durch ein, zwei oder drei $C_1$-$C_4$-Alkoxygruppen substituiertes Phenyl,

- durch ein, zwei oder drei $C_1$-$C_4$-Halogenalkylgruppen substituiertes Phenyl,
- durch ein oder zwei Nitrogruppen substituiertes Phenyl,
- durch ein, zwei oder drei Phenoxygruppen substituiertes Phenyl,
- durch Halogen und $C_1$-$C_4$-Alkyl zwei- oder dreifach substituiertes Phenyl,
- durch Halogen und $C_1$-$C_4$-Alkoxy zwei- oder dreifach substituiertes Phenyl,
- durch Halogen und $C_1$-$C_4$-Halogenalkyl zwei- oder dreifach substituiertes Phenyl,
- durch Halogen und Nitro zwei- oder dreifach substituiertes Phenyl,
- durch Halogen und Phenoxy zwei- oder dreifach substituiertes Phenyl,
- durch $C_1$-$C_4$-Alkyl und $C_1$-$C_4$-Alkoxy zwei- oder dreifach substituiertes Phenyl,
- durch $C_1$-$C_4$-Alkyl und $C_1$-$C_4$-Halogenalkyl zwei- oder dreifach substituiertes Phenyl,
- durch $C_1$-$C_4$-Alkyl und Nitro zwei- oder dreifach substituiertes Phenyl,
- durch $C_1$-$C_4$-Alkyl und Phenoxy zwei- oder dreifach substituiertes Phenyl,
- durch $C_1$-$C_4$-Alkoxy und $C_1$-$C_4$-Halogenalkyl zwei- oder dreifach substituiertes Phenyl,
- durch $C_1$-$C_4$-Alkoxy und Nitro zwei- oder dreifach substituiertes Phenyl,
- durch $C_1$-$C_4$-Alkoxy und Phenoxy zwei- oder dreifach substituiertes Phenyl,
- durch $C_1$-$C_4$-Halogenalkyl und Nitro zwei- oder dreifach substituiertes Phenyl,
- durch $C_1$-$C_4$-Halogenalkyl und Phenoxy zwei- oder dreifach substituiertes Phenyl,
- durch Nitro und Phenoxy zwei- oder dreifach substituiertes Phenyl,
- durch Halogen, $C_1$-$C_4$-Alkyl und $C_1$-$C_4$-Alkoxy dreifach substituiertes Phenyl,
- durch Halogen, $C_1$-$C_4$-Alkyl und $C_1$-$C_4$-Halogenalkyl dreifach substituiertes Phenyl
- durch Halogen, $C_1$-$C_4$-Alkyl und Nitro dreifach substituiertes Phenyl
- durch Halogen, $C_1$-$C_4$-Alkyl und Phenoxy dreifach substituiertes Phenyl
- durch Halogen, $C_1$-$C_4$-Alkoxy und $C_1$-$C_4$-Halogenalkyl dreifach substituiertes Phenyl
- durch Halogen, $C_1$-$C_4$-Alkoxy und Nitro dreifach substituiertes Phenyl
- durch Halogen, $C_1$-$C_4$-Alkoxy und Phenoxy dreifach substituiertes Phenyl
- durch Halogen, $C_1$-$C_4$-Halogenalkyl und Nitro dreifach substituiertes Phenyl
- durch Halogen, $C_1$-$C_4$-Halogenalkyl und Phenoxy dreifach substituiertes Phenyl
- durch Halogen, Nitro und Phenoxy dreifach substituiertes Phenyl
- durch $C_1$-$C_4$-Alkyl, $C_1$-$C_4$-Alkoxy und $C_1$-$C_4$-Halogenalkyl dreifach substituiertes Phenyl
- durch $C_1$-$C_4$-Alkyl, $C_1$-$C_4$-Alkoxy und Nitro dreifach substituiertes Phenyl
- durch $C_1$-$C_4$-Alkyl, $C_1$-$C_4$-Alkoxy und Phenoxy dreifach substituiertes Phenyl
- durch $C_1$-$C_4$-Alkyl, $C_1$-$C_4$-Halogenalkyl und Nitro dreifach substituiertes Phenyl
- durch $C_1$-$C_4$-Alkyl, $C_1$-$C_4$-Halogenalkyl und Phenoxy dreifach substituiertes Phenyl
- durch $C_1$-$C_4$-Alkyl, Nitro und Phenoxy dreifach substituiertes Phenyl
- durch $C_1$-$C_4$-Alkoxy, $C_1$-$C_4$-Halogenalkyl und Nitro dreifach substituiertes Phenyl
- durch $C_1$-$C_4$-Alkoxy, $C_1$-$C_4$-Halogenalkyl und Phenoxy dreifach substituiertes Phenyl
- durch $C_1$-$C_4$-Halogenalkyl, Nitro und Phenoxy dreifach substituiertes Phenyl.

Besonders bevorzugt sind die Verbindungen I und II, in denen A für 4-Fluorphenyl, 4-Chlorphenyl, Phenyl, tert.-Butyl, sec.-Butyl, iso-Butyl und iso-Propyl und B für substituierte Phenyle, wie 2-Fluorphenyl, 4-Fluorphenyl, 2-Chlorphenyl, 4-Chlorphenyl, 2-Bromphenyl, 4-Bromphenyl, 2,4-Difluorphenyl, 2,4-Dichlorphenyl, 2,3-Dichlorphenyl, 2,5-Dichlorphenyl, 2,6-Dichlorphenyl, 3,4-Dichlorphenyl und 3,4-Dichlorphenyl steht.

Der Substituent X in Formel II bedeutet eine unter den Reaktionsbedingungen eliminierbare Gruppe wie Acetoxy, Pivaloyloxy, Benzoyloxy, Halogen, wie Chlor und Brom, Alkyl- oder Arylsulfonyloxy, Trialkyl silyloxy, Phenyldialkylsilyloxy und Diphenylalkylsilyloxy, bevorzugt Chlor, Brom, Acetoxy, Benzoyloxy, Trialkylsilyloxy, Tosyloxy und Mesyloxy, besonders bevorzugt Chlor, Brom, Trimethylsilyloxy, Acetoxy und Mesyloxy.

Die Substituenten $A'$, $B'$ und $X'$ in den Ketonen der Formel $II'$ haben folgende Bedeutungen:

$A'$ Phenyl und 4-Fluorphenyl, bevorzugt 4-Fluorphenyl

$B'$ 2-Fluorphenyl, 2-Chlorphenyl, 2-Bromphenyl und 2-Trifluormethylphenyl, bevorzugt 2-Chlorphenyl, 2-Fluorphenyl, 2-Trifluormethylphenyl, besonders bevorzugt 2-Chlorphenyl und

$X'$ Hydroxy oder eine Abgangsgruppe wie Acetoxy, Pivaloyloxy, Benzoyloxy, Halogen, wie Chlor und Brom, Alkyl- oder Arylsulfonyloxy, Trialkylsilyloxy, Phenyldialkylsilyloxy und Diphenylalkylsilyloxy, bevorzugt Chlor, Brom, Acetoxy, Benzoyloxy, Trialkylsilyloxy, Tosyloxy und Mesyloxy, besonders bevorzugt Chlor, Brom, Trimethylsilyloxy, Acetoxy und Mesyloxy,

mit der Maßgabe, daß $B'$ nicht für 2-Chlorphenyl steht, wenn $A'$ Phenyl bedeutet.

Als bevorzugte Ketone II und $II'$ seien genannt:

1-(4-Fluorphenyl)-2-(2-chlorphenyl)-2-hydroxy-ethan-1-on

1-(4-Fluorphenyl)-2-(2-chlorphenyl)-2-trimethylsilyloxy-ethan-1-on
1-(4-Fluorphenyl)-2-(2-chlorphenyl)-2-chlor-ethan-1-on
1-(4-Fluorphenyl)-2-(2-chlorphenyl)-2-acetoxy-ethan-1-on
1-(4-Fluorphenyl)-2-(2-chlorphenyl)-2-methansulfonyloxy-ethan-1-on
1-(4-Fluorphenyl)-2-(2-chlorphenyl)-2-toluolsulfonyloxy-ethan-1-on
1-(4-Chlorphenyl)-2-(2-chlorphenyl)-2-hydroxy-ethan-1-on
1-(4-Chlorphenyl)-2-(2-chlorphenyl)-2-trimethylsilyloxy-ethan-1-on
1-(4-Chlorphenyl)-2-(2-chlorphenyl)-2-chlor-ethan-1-on
1-(4-Chlorphenyl)-2-(2-chlorphenyl)-2-acetoxy-ethan-1-on
1-(4-Chlorphenyl)-2-(2-chlorphenyl)-2-methansulfonyloxy-ethan-1-on
1-(4-Chlorphenyl)-2-(2-chlorphenyl)-2-toluolsulfonyloxy-ethan-1-on
1-Phenyl-2-hydroxy-3,3-dimethyl-butan-1-on
1-(4-Fluorphenyl)-2-hydroxy-3,3-dimethyl-butan-1-on
1-(4-Chlorphenyl)-2-hydroxy-3,3-dimethyl-butan-1-on
1-Phenyl-2-chlor-3,3-dimethyl-butan-1-on
1-(4-Fluorphenyl)-2-chlor-3,3-dimethyl-butan-1-on
1-(4-Chlorphenyl)-2-chlor-3,3-dimethyl-butan-1-on
1-Phenyl-2-brom-3,3-dimethyl-butan-1-on
1-(4-Fluorphenyl)-2-brom-3,3-dimethyl-butan-1-on
1-(4-Chlorphenyl)-2-brom-3,3-dimethyl-butan-1-on
1-Phenyl-2-toluolsulfonyloxy-3,3-dimethyl-butan-1-on
1-(4-Fluorphenyl)-2-toluolsulfonyloxy-3,3-dimethyl-butan-1-on
1-(4-Chlorphenyl)-2-toluolsulfonyloxy-3,3-dimethyl-butan-1-on
1-Phenyl-2-methansulfonyloxy-3,3-dimethyl-butan-1-on
1-(4-Fluorphenyl)-2-methansulfonyloxy-3,3-dimethyl-butan-1-on
1-(4-Chlorphenyl)-2-methansulfonyloxy-3,3-dimethyl-butan-1-on
1-Phenyl-2-trimethylsilyloxy-3,3-dimethyl-butan-1-on
1-(4-Fluorphenyl)-2-trimethylsilyloxy-3,3-dimethyl-butan-1-on
1-(4-Chlorphenyl)-2-trimethylsilyloxy-3,3-dimethyl-butan-1-on
1-Phenyl-1-hydroxy-3,3-dimethyl-butan-2-on
1-(4-Fluorphenyl)-1-hydroxy-3,3-dimethyl-butan-2-on
1-(4-Chlorphenyl)-1-hydroxy-3,3-dimethyl-butan-2-on
1-Phenyl-1-chlor-1-hydroxy-3,3-dimethyl-butan-2-on
1-(4-Fluorphenyl)-1-chlor-1-hydroxy-3,3-dimethyl-butan-2-on
1-(4-Chlorphenyl)-1-chlor-1-hydroxy-3,3-dimethyl-butan-2-on
1-Phenyl-1-brom-1-hydroxy-3,3-dimethyl-butan-2-on
1-(4-Fluorphenyl)-1-brom-1-hydroxy-3,3-dimethyl-butan-2-on
1-(4-Chlorphenyl)-1-brom-1-hydroxy-3,3-dimethyl-butan-2-on
1-Phenyl-1-toluolsulfonyloxy-1-hydroxy-3,3-dimethyl-butan-2-on
1-(4-Fluorphenyl)-1-toluolsulfonyloxy-1-hydroxy-3,3-dimethyl-butan-2-on
1-(4-Chlorphenyl)-1-toluolsulfonyloxy-1-hydroxy-3,3-dimethyl-butan-2-on
1-Phenyl-1-methansulfonyloxy-1-hydroxy-3,3-dimethyl-butan-2-on
1-(4-Fluorphenyl)-1-methansulfonyloxy-1-hydroxy-3,3-dimethyl-butan-2-on
1-(4-Chlorphenyl)-1-methansulfonyloxy-1-hydroxy-3,3-dimethyl-butan-2-on
1-Phenyl-1-trimethylsilyloxy-1-hydroxy-3,3-dimethyl-butan-2-on
1-(4-Fluorphenyl)-1-trimethylsilyloxy-1-hydroxy-3,3-dimethyl-butan-2-on
1-(4-Chlorphenyl)-1-trimethylsilyloxy-1-hydroxy-3,3-dimethyl-butan-2-on

Als bevorzugte in 2-Position Z-substituierte 1-Hydroxymethyl-oxirane I seien genannt:

(Z)-1-Hydroxy-2-(4-fluorphenyl)-3-(2-chlorphenyl)-2,3-epoxypropan
(Z)-1-Hydroxy-2-(4-fluorphenyl)-3-(2-trifluormethylphenyl)-2,3-epoxypropan
(Z)-1-Hydroxy-2-(4-fluorphenyl)-3-(2-bromphenyl)-2,3-epoxypropan
(Z)-1-Hydroxy-2-(4-fluorphenyl)-3-(2-fluorphenyl)-2,3-epoxypropan
(Z)-1-Hydroxy-2-Phenyl-3-(2-chlorphenyl)-2,3-epoxypropan
(Z)-1-Hydroxy-2-Phenyl-3-(2-trifluormethylphenyl)-2,3-epoxypropan
(Z)-1-Hydroxy-2-Phenyl-3-(2-bromphenyl)-2,3-epoxypropan
(Z)-1-Hydroxy-2-Phenyl-3-(2-fluorphenyl)-2,3-epoxypropan

Die in 2-Position Z-substituierten (R,S)-1′-Hydroxymethyl-oxirane I sind wertvolle Zwischenprodukte zur Synthese von antimykotisch und fungizid (EP-A-94 564) sowie pflanzenwachstumsregulierend (DE-A-3737888) wirksamen Azolylmethyloxiranen

Beispiel

Herstellung von (Z)-1-Hydroxy-2-(4-fluorphenyl)-3-(2-chlorphenyl)-2,3-epoxypropan

8,3 g (25 mmol) 1-(4-Fluorphenyl)-2-(trimethylsilyloxy)-2-(2-chlorphenyl)-ethan-1-on wurden in Dimethylsulfoxid gelöst und zu 75 mmol frisch bereitetem Trimethyloxosulfonium-methylid bei 30 bis 40° C getropft, 5 Stunden bei 35 bis 40° C nachgerührt und anschließend auf Eiswasser gegeben. Nach Extraktion mit Essigester und Einengen der org. Phase erhält man 7,6 g eines Öls bestehend zu 41 % aus (Z)-2-(4-Fluorphenyl)-3-(2-chlorphenyl)-2-(hydroxymethyl)-oxiran gemäß quantitativer GC-Analyse. Das E-Isomere war nicht nachweisbar.

Das Produkt wurde durch Chromatographie an Kieselgel mit Cyclohexan/Essigester (5:1) gereinigt und $^{1}$H-NMR-spektroskopisch identifiziert.

$^{1}$H-NMR ($CDCl_3$/TMS) in ppm: δ 1,6 s, breit (1H); 3,5 d (1H); 3,95 d (1H); 4,15 s (1H); 7,0-8,0 m (8H).

Das Ausgangsprodukt 1-(4-Fluorphenyl)-2-(trimethylsilyloxy)-2-(2-chlorphenyl)-ethan-1-on kann wie folgt erhalten werden:

Herstellung von 4-Fluor-2'-chlorbenzoin

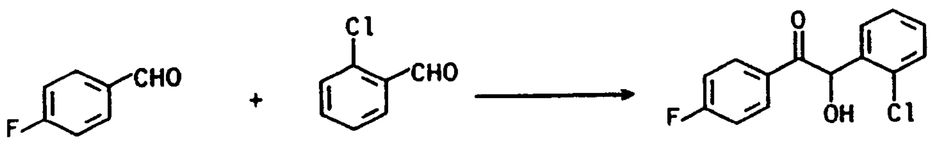

Eine Mischung aus 14,1 g (0,1 mol) 2-Chlorbenzaldehyd, 12,4 g (0,1 mol) 4-Fluorbenzaldehyd, 2,5 g (0,01 mol) 3-Ethyl-5-(2-hydroxyethyl)-4-methylthiazoliumbromid, 6,1 g (0,06 mol) Triethylamin, 60 ml Ethanol und 3 g Molekularsieb (3A) wurde unter Stickstoffatmosphäre 90 Min unter Rückfluß erhitzt, anschließend in Eiswasser gegossen und mit Methylenchlorid extrahiert. Die organische Phase wurde mit Natriumhydrogencarbonatlösung und Wasser gewaschen, mit Natriumsulfat getrocknet und eingeengt. Man erhielt 23,8 eines Öls, das nach $^{1}$H-NMR-Analyse zu 70 % aus 4-Fluor-2'-chlorbenzoin bestand. Dieses ließ sich aus Cyclohexan umkristallisieren.

$^{1}$H-NMR ($CDCl_3$/TMS) in ppm: δ 4,6 s, breit (1H); 6,35 s (1H); 7,0-8,0 m (8H).

Herstellung von 1-(4-Fluorphenyl)-2-(trimethylsilyloxy)-2-(2-chlorphenyl)-ethan-1-on

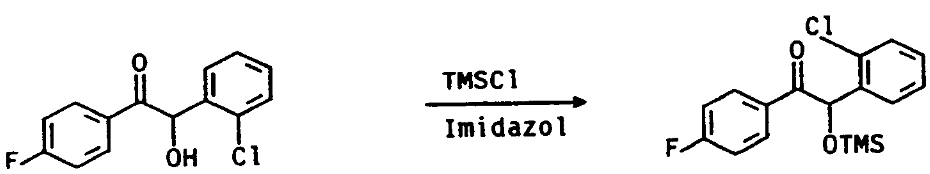


Zu einer Lösung von 53,2 g (0,2 mol) 4-Fluor-2-chlorbenzoin und 27,2 g (0,4 mol) Imidazol in 200 ml Dimethylformamid tropfte man bei Raumtemperatur innerhalb von 30 Min. 28,2 ml Trimethylsilylchlorid. Nach 6 Std. bei Raumtemperatur wurde der Ansatz auf 1 l Wasser gegossen und zweimal mit Pentan extrahiert. Die Pentanphase wurde getrocknet und eingeengt. Man erhielt 66 g (98 %) eines gelben Öls.

$^{1}$H-NMR ($CDCl_3$, TMS) in ppm: δ 0,13 s (9H); 6,4 s (1H); 7,0-8,0 m (8H).

**Ansprüche**

1. Verfahren zur Herstellung von in 2-Position Z-substituierten 1-Hydroxymethyl-oxiranen der allgemeinen Formeln I und ent-I,

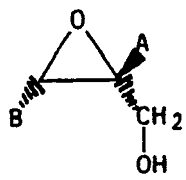

(I)

und

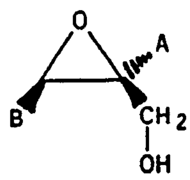

(ent-I)

in der die Substituenten

A, B unabhängig voneinander $C_1$-$C_4$-Alkyl, Phenyl, Naphthyl, Diphenyl oder durch ein bis drei Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl

bedeuten, dadurch gekennzeichnet, daß man Ketone der allgemeinen Formel II

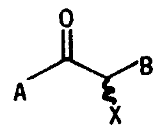

(II),

in der A und B die oben genannten Bedeutungen haben und X für eine unter den Reaktionsbedingungen eliminierbare Gruppe steht, mit Trimethylsulfoniummethylsulfat und einer geeigneten Base oder mit Dimethyloxosulfonium-methylid umsetzt.

2. Ketone der allgemeine Formel II′

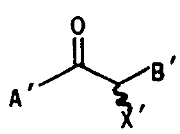

(II′),

in der die Substituenten

A′ Phenyl oder 4-Fluorphenyl,

B′ 2-Fluorphenyl, 2-Chlorphenyl, 2-Bromphenyl oder 2-Trifluormethylphenyl und

X′ Hydroxy oder eine Abgangsgruppe

bedeuten, mit der Maßgabe, daß B′ nicht für 2-Chlorphenyl steht, wenn A′ Phenyl bedeutet.